Europäisches Patentamt

European Patent Office

Office européen des brevets

(19)

(11) Veröffentlichungsnummer: **0 338 524 B1**

# (12) EUROPÄISCHE PATENTSCHRIFT

(45) Veröffentlichungstag der Patentschrift: **25.01.95**

(51) Int. Cl.[6]: **A63B 33/00**, A61F 9/02

(21) Anmeldenummer: **89106984.1**

(22) Anmeldetag: **19.04.89**

(54) **Taucehermaske, Schwimmbrille, Windschutzbrille oder dergleichen mit Sehkorrektiv sowie Verfahren zu dessen Herstellung.**

(30) Priorität: **22.04.88 DE 3813507**

(43) Veröffentlichungstag der Anmeldung:
**25.10.89 Patentblatt 89/43**

(45) Bekanntmachung des Hinweises auf die
Patenterteilung:
**25.01.95 Patentblatt 95/04**

(84) Benannte Vertragsstaaten:
**AT BE CH DE ES FR GB GR IT LI LU NL SE**

(56) Entgegenhaltungen:
**EP-A- 0 182 503**
**DE-A- 2 809 205**
**US-A- 2 361 589**
**US-A- 3 944 345**

(73) Patentinhaber: **Hala, Ralf**
**An der Säge 12**
**D-88161 Lindenberg (DE)**

(72) Erfinder: **Hala, Ralf**
**An der Säge 12**
**D-88161 Lindenberg (DE)**

EP 0 338 524 B1

Rank Xerox (UK) Business Services
(3.10/3.09/3.3.3)

EP 0 338 524 B1

**Beschreibung**

Die Erfindung betrifft eine Tauchmaske, eine Schwimmbrille eine Windschutzbrille oder dergleichen mit wenigstens einer Sichtscheibe, an deren Oberfläche weingstens ein optisch aktives Element als Sehkorrectiv mit Hilfe einer transparenten Harzschicht befestigt ist.

Tauchermasken oder Schutzbrillen dieser Art sind grundsätzlich bekannt und beispielsweise in der DE-A 28 09 205 und auch in der FR-A 1 374 010 beschrieben.

Bei den vorbekannten Tauchmasken, Schwimmbrillen oder dergleichen sind optische Linsensysteme, Sehkorrektive oder speziell für diesen Zweck angefertigte Systeme, die Plankonkav oder Plankonvex ausgebildet sind, mittels einer dünnen Harzschicht auf die Sichtscheibe der Maske bzw. Brille geklebt.

Die Harzschicht zwischen der planen Vorderfläche der Linsensysteme oder Sehkorrektive und der planen Sichtscheibe der Maske oder Brille ist somit plan bzw. planparallel ausgebildet.

Als Harze zum Befestigen der optischen Systeme oder Elemente welche als Sehkorrektiv wirken, können natürliche oder künstliche Harze verwendet werden. Als Beispiel für ein natürliches Harz wird Kanada Balsam und als Beispiele für synthetische Harze werden thermoplastische Polyester-Harze oder auch thermoplastische Silikonharze genannt. Auch Zweikomponenten-Harze können eingesetzt werden.

Zum Stande der Technik wird noch auf das DE-GM 19 09 084 verwiesen, in welchem ein Augenschutzglas für Schutzbrillen oder dergleichen beschrieben ist, bei dem die dem Auge zugewandte Seite aus einer Kunststoffschicht und die dem Auge abgewandte Seite aus einer Glasscheibe besteht. Auch in dieser Veröffentlichung ist angegeben, daß zur Korrektur von Augenfehlern die Kunststoffscheibe und/oder die Glasschicht auf beiden Seiten unterschiedlich gewölbt sein kann.

Die vorbekannten Ausführungsformen derartiger Tauchmasken, Schwimmbrillen, Windschutzbrillen oder dergleichen weisen erhebliche Nachteile auf. Diese Nachteile bestehen zum einen in dem verhältnismäßig hohen Preis der erforderlichen Linsen oder Linsensysteme zum anderen in der Schwierigkeit, die erforderliche Korrektion den individuellen Gegebenheiten anzupassen. Insbesondere die Korrektur des Astigmatismus mixtus kann nur durch einen sogenannte Sattelzylinderfläche erreicht werden. Diese Sattelzylinderflächen sind technisch nur schwierig herstellbar und folglich teuer. Desweiteren haben plankonkave oder plankonvexe Linsen gegenüber punktuell abbildenden Brillengläsern erheblich schlechtere Abbildungseigenschaften. Insbesondere die periphere Sehschärfe nimmt deutlich ab.

Bei Verwendung von besonderen Halterungen in Verbindung mit Korrekturlinsen ist bei Druck- oder Temperaturänderungen eine einwandfreie Haftung z.B. durch Adhäsion nicht gewährleistet. Sichtbehinderungen durch Beschlagen treten bei diesen Lösungen verstärkt auf.

Der Erfindung liegt daher die Aufgabe zugrunde, die vorbekannten Tauchermasken, Schwimmbrillen, Windschutzbrillen oder dergleichen dahingehend weiter zu verbessern, das sie zum einen leichter herstellbar und somit billiger werden und zum anderen leichter auf die individuellen Gegebenheiten einstellbar sind.

Zur Lösung dieser Aufgaben wird von einer Tauchermaske, Schwimmbrille, Windschutzbrille oder dergleichen mit wenigstens einer planen Sichtscheibe, an deren Oberfläche sich wenigstens ein optisch aktives Element das als Sehkorrektiv ausgebildet ist befindet, welches mit Hilfe einer transparenten Harzschicht befestigt ist, oder die tranparente Harzschicht selbst bildet, ausgegangen.

Die Lösung besteht erfindungsgemäß darin, daß die Harzschicht, deren Oberfläche nicht an der Sichtscheibe der Maske oder Brille anliegt, gekrümmt ausgebildet ist, und somit als Sehkorrektiv wirkt. Dabei kann die Harzschicht das einzig vorhandene Sehkorrektiv sein oder es kann zusätzlich eine Linse, ein Brillenglas oder auch ein zusammengesetztes optisches System vorhanden sein.

Die Einbeziehung der Harzschicht als Sehkorrektiv bietet einerseits die Möglichkeit zur angestrebten Verbilligung andererseits aber auch den Vorteil, daß die Dezentration (Lage des optischen Mittelpunktes vor dem Auge) an die individuellen Gegebenheiten, sowie die Korrektion prismatischer Fehlsichtigkeit leicht vorgenommen werden kann.

Bei Anwendung eines Brillenglases, welches erfindungsgemäß mit Hilfe einer wenigen Millimeter dicken Harzschicht von innen auf der bzw. den Frontscheiben der Tauch- oder Schwimmbrille dauerhaft befestigt ist, befindet sich die Harzschicht zwischen der Frontscheibe und der objektseitigen Oberfläche des Brillenglases, wobei die Harzschicht einen möglichst niederen Brechungsindex aufweisen soll.

Die objektseitige Flächenbrechkraft des Brillenglases verliert somit einen Teil ihrer dioptrischen Wirkung. Die objektseitige Flächenbrechkraft des verwendeten Brillenglases berechnet man wie folgt.

Fl = Flächenbrechkraft gegen Luft

Fsl = Flächenbrechkraft gegen Trägerschicht

ngl = Brechnungsindex des Brillenglases

nsl = Brechnungsindex der Harzschicht

r    = Radius der objektseitigen Glaskurve des Brillenglases

$$\frac{Fl}{Fsl} = \frac{\dfrac{ngl - 1}{r}}{\dfrac{ngl - nsl}{r}} \quad ; \quad Fsl = \frac{(ngl-nsl)\cdot Fl}{ngl - 1}$$

Der Brechkraftverlust der objektseitigen Fläche muß durch entsprechende Auswahl eines geeigneten Brillenglases berücksichtigt werden. Somit kann zwar die punktuelle Abbildung in der Regel nicht exakt eingehalten werden, die Abbildungseigenschaften sind aber besser als die von Plankonvex- oder Plankonkavlinsen.

Zur Herstellung der vorgeschlagenen Masken, Brillen und dergleichen wird vorteilhafterweise so verfahren, daß auf die innere oder äußere Oberfläche der Sichtscheibe eine transparente Schicht eines zunächst flüssigen oder wenigstens verformbaren Harzes aufgebracht, in dieses ein individuell ausgesuchtes optisches Element oder System eingedrückt und das Harz sodann entweder bei fortdauerndem Kontakt mit dem optischen Element oder System oder nach dessen Wiederentfernen erstarren gelassen wird.

Dieses Verfahren ermöglicht es einerseits, die vorgeschlagenen Masken oder Brillen in wirtschaftlicher Weise herzustellen. Desweiteren ermöglicht das beschriebene Verfahren aber eine leichte Dezentrierung des Brillenglases, also eine Verschiebung der Lage des optischen Mittelpunktes vor dem Augendrehpunkt.

Bei herkömmlichen Verfahren muß die Dezentrierung des optischen Mittelpunktes gegenüber der geometrischen Mitte der Glasform vor dem Randen des Glases festgelegt werden. Insbesondere bei großen Sichtscheiben sind deshalb große Rohglasdurchmesser zur exakten Dezentration notwendig, die erheblich größer sind als die größte Ausdehnung der Sichtscheibe. Da derart große Rohglasdurchmesser teilweise nicht erhältlich sind, muß oft auf Gläser mit prismatischer Komponente zurückgegriffen werden, was erhebliche Mehrkosten und eine Gewichtserhöhung verursacht.

Im Gegensatz hierzu kann bei dem beschriebenen Verfahren eine Dezentrierung durch einfaches Verkippen des Brillenglases, einer Hilfsverglasungsscheibe oder des formgebenden Werkzeugs erzielt werden. Dasselbe gilt auch für die Erzielung prismatischer Korrektionswirkungen.

In weiterer Ausgestaltung der Erfindung wird vorgeschlagen, daß die erwähnte Harzschicht aus verschiedenen Harzsorten mit unterschiedlichen Brechungsindices besteht, wodurch unterschiedliche dioptrische Wirkungen erzielt werden können. Die verschiedenen Harze können in Form von mehreren Schichten mit jeweils unterschiedlichem Brechnungsindex punktuell oder graduell aufgebracht und miteinander verbunden werden.

Desweiteren ist es möglich, in die Harzschicht Planstützscheiben oder Hilfsverglasungsscheiben mit unterschiedlichen Durchbiegungen einzubetten. Hierdurch ergibt sich eine der zuvor erwähnten Möglichkeiten der Dezentration, die darin besteht, daß bei der Positionierung der optischen Elemente, Systeme, Stütz- oder Hilfsverglasungsscheiben diese gegenüber der Sehachse verkippt werden. (Das menschliche Auge ist von annähernd kugelförmiger Gestalt, stellt jedoch kein im Sinne der geometrischen Optik exakt zentriertes System dar. Als optische Achse des Auges, auch Sehachse genannt, wird daher das Lot auf die Hornhautvorderfläche festgelegt, welches durch die Mitte der Eintrittspupille geht).

Da es bei den vorgeschlagenen Sehhilfen möglich ist, auf Brillengläser, zusätzliche Linsensysteme und dergleichen ganz zu verzichten, ergibt sich eine weitere Vereinfachung des Herstellungsvorgangs dadurch, daß nach dem Aufbringen der Harzschicht diese im noch verformbaren Zustand mit Hilfe eines Preßstempels in die gewünschte Oberflächenform gebracht wird. Dieser Preßstempel kann aus Stahl oder einem anderen geeigneten Werkstoff bestehen und folglich nahezu beliebig oft verwendet werden.

Als weitere Vorteile der vorgeschlagenen Maske oder Brille sowie des vorgeschlagenen Herstellungsverfahrens ist zu erwähnen, daß die Korrektur des Astigmatismus mixtus wie die Korrektur aller anderen Astigmatismen möglich ist, insbesondere, wenn innentorische Brillengläser verwendet werden und die Brechungsindices von Brillenglas und Harzschicht möglichst stark differieren.

Nach Auswahl der entsprechenden Gläser wird erfindungsgemäß entweder die Frontscheibe flächendeckend mit einer ausreichend dicken Schicht des Harzes versehen und das Brillenglas in seiner optisch korrekten Position (Zentrierung, Nahteilhöhe, Pupillenabstand usw.) aufgebracht, oder die Harzschicht auf

3

EP 0 338 524 B1

die geometrische Form beschränkt.

Die Erfindung wird im folgenden Anhand der beigefügten Zeichnung erläutert.

In den Zeichnungen 1 bis 3 sind jeweils Längsschnitte des Maskenkörpers mit verschiedenen eingesetzten Korrektursystemen dargestellt.

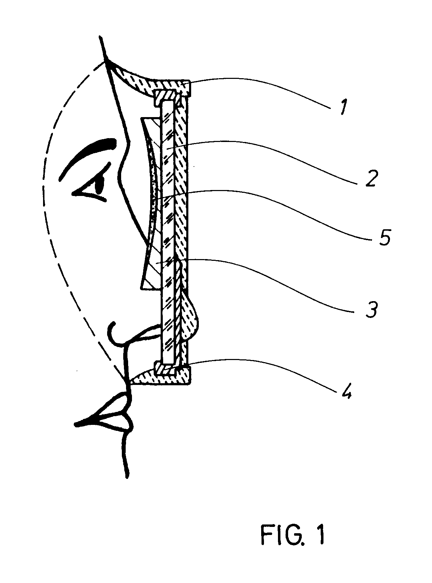

Figur 1 zeigt eine Schutzmaske, bestehend aus dem Maskenkörper (1) mit Sichtscheibe (2) und Sichtscheibenhalterung (4).

Auf der Rückseite der Sichtscheibe (2) ist eine Harzschicht (3), die selbst als Aufnahme für ein optisch wirksames Element, z.B. ein Brillenglas dient.

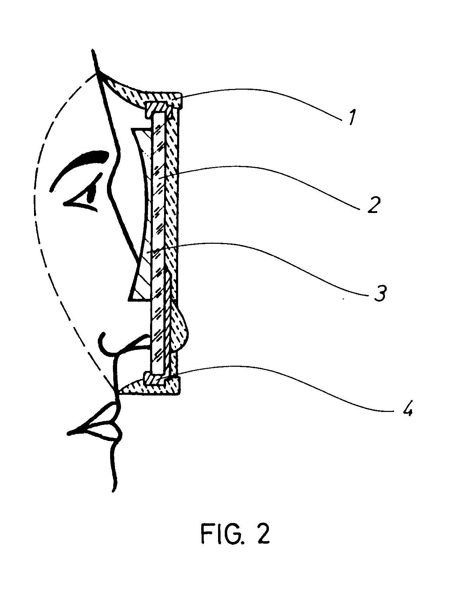

Figur 2 zeigt ebenfalls eine Schutzmaske, die aus dem Maskenkörper (1) besteht, in den eine Sichtscheibe (2) nebst Sichtscheibenhalterung (4) eingesetzt ist. Auf der Rückseite der Sichtscheibe (2) befindet sich die Harzschicht (3) die nun selbst als Sehkorrektiv ausgebildet ist.

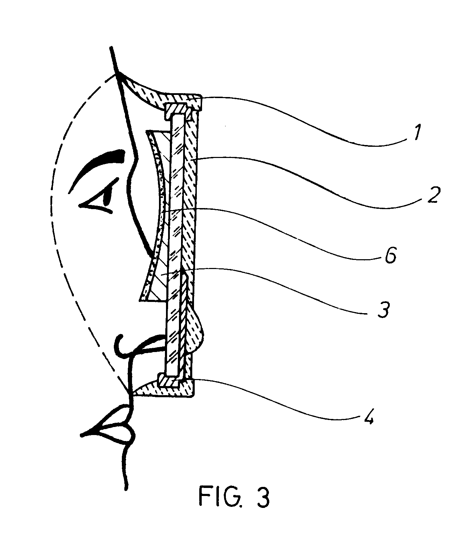

Figur 3 zeigt eine Schutzmaske, die aus dem Maskenkörper (1) besteht, in welchem eine Sichtscheibe (2) umgeben von einer Sichtscheibenhalterung (4) gehalten wird. Auf der Rückseite der Sichtscheibe (2) befindet sich die Harzschicht (3), in der die Hilfsverglasungsscheibe (6) ohne dioptrische Wirkung eingebettet ist.

Bezugszeichenliste

1 Maskenkörper
2 Sichtscheibe
3 Harzschicht
4 Sichtscheibenhalterung
5 optisches Element
6 Hilfsverglasungsscheibe

**Patentansprüche**

1. Tauchermaske, Schwimmbrille, Windschutzbrille oder dergleichen mit wenigstens einer planen Sichtscheibe (2) und wenigstens einer daran angebrachten optisch aktiven Einrichtung, dadurch gekennzeichnet, daß die optisch aktive Einrichtung eine transparente Harzschicht (3) aufweist, eine Oberfläche gegen die Sichtscheibe (2) anliegt und die andere Oberfläche der Harzschicht gekrümmt ausgebildet ist, wodurch die Harzschicht als optisch aktives Sehkorrektiv wirkt.

2. Tauchermaske, Schwimmbrille, Windschutzbrille oder dergleichen nach Anspruch 1 dadurch gekennzeichnet, daß die an der Sichtscheibe (2) befestigte Harzschicht als einziges optisch aktive Sehkorrektiv vorhanden ist.

3. Tauchermaske, Schwimmbrille, Windschutzbrille oder dergleichen nach Anspruch 1 dadurch gekennzeichnet, daß an der Oberfläche der Sichtscheibe (2) ein optisches Element (5) als Sehkorrektiv mit Hilfe der transparenten Harzschicht (3) befestigt ist.

4. Verfahren zum Herstellen einer Tauchermaske oder dergleichen nach Anspruch 1 oder 3 dadurch gekennzeichnet, daß auf die innere oder äußere Oberfläche einer planen Sichtscheibe (2) eine transparente Schicht eines zunächst flüssigen oder wenigstens verformbaren Harzes aufgebracht, in dieses ein gekrümmtes individuell ausgesuchtes optisches Element oder System eingedrückt, dieses optische Element oder System gegenüber der Sehachse bzw. der optischen Achse verkippt wird und das Harz sodann entweder bei fortlaufendem Kontakt mit dem optischen Element oder System oder nach dessen Wiederentfernen erstarren gelassen wird.

5. Verfahren nach Anspruch 4 dadurch gekennzeichnet, daß als Harzschicht verschiedene Harzsorten mit unterschiedlichen Brechungsindices zur Erzielung unterschiedlicher dioptrischer Wirkungen aufgebracht werden.

6. Verfahren nach Anspruch 4 und 5 dadurch gekennzeichnet, daß die verschiedenen Harze in Form von mehreren Schichten mit jeweils unterschiedlichem Brechungsindex partiell, punktuell oder graduell aufgebracht und miteinander verbunden werden.

4

**7.** Verfahren nach Anspruch 4, 5 oder 6 dadurch gekennzeichnet, daß in die Harzschicht (3) Planstütz- oder Hilfsverglasungsscheiben mit unterschiedlichen Durchbiegungen eingebettet werden.

## Claims

**1.** A diving mask, swimming goggle, wind goggle and the like with at least one plain face plate (2) and at least one thereon attached optical efficacious device characterised in that the optical efficacious device has a transparent resin layer (3), one surface adjacent to the face plate, the other surface is curved, whereby the resin layer operates as optical efficacious vision corrector.

**2.** A diving mask, swimming goggle, wind goggle and the like according to claim 1, characterised in that the resin layer is as single optical efficacious device attached to the surface of the face plate.

**3.** A diving mask, swimming goggle, wind goggle and the like according to claim 1, characterised in that on the surface of the face plate an optical element (5) is attached as vision corrcetor by the transparent resin layer.

**4.** Procedure for manufacturing a diving mask or the like according to claim 1 or 3 , characterised in that the inner or outer surface of a plain face plate (2) is applied to a transparent layer of an initially liquid or at least mouldable resin in which a curved, individually selected optical element or system is pressed, this optical element or system is tilt against the visual axis and the optical axis respectively, then the resin will be solidified either under continuous contact or after removing the optical element or system.

**5.** Procedure for manufacturing a diving mask or the like according to claim 4, characterised in that as resin layer different kinds of resins with different refraction indices are applied to obtain different dioptrical effects.

**6.** Procedure for manufacturing a diving mask or the like according to claim 4 and 5, characterised in that several layers of different resins with different refraction indices are applied and connected together partially, punctually or gradually.

**7.** Procedure for manufacturing a diving mask or the like according to claim 4,5 or 6, characterised in that into the resin layer(3) demonstration lenses with different curves are embedded.

## Revendications

**1.** Masque de plongée, lunettes de natation, lunettes de protection ou analogue comportant au moins une vitre plane, ladite étant équipée d'au moins un dispositif optique actif, caractérisé en ce que le dispositif optique actif présente une couche de résine dont l'une des faces est appliquée sur la vitre, et dont l'autre face est de forme courbe, cette couche de résine jouant un rôle de correction de la vue.

**2.** Masque de plongée, lunettes de natation, lunettes de protection ou analogue selon la revendication 1, caractérisé en ce que la couche de résine appliquée sur la vitre constitue l'unique dispositif de correction de la vue.

**3.** Masque de plongée, lunettes de natation, lunettes de protection ou analogue selon la revendication 1, caractérisé en ce qu'un dispositif de correction de la vue est fixé à la vitre par l'intermédiaire de la couche de résine.

**4.** Procédé d'obtention d'un masque de plongée ou analogue selon la revendication 1 ou 3, caractérisé en ce que sur une couche de résine de prime abord liquide ou au moins maléable déposée sur la surface intérieur ou extérieur de la vitre, on applique un élément ou un système optique adapté qui peut être incliné par raport à l'axe visuel ou optique et que l'on laisse en place ou non pendant la solidification de la résine.

**5.** Procédé d'obtention selon la revendication 4 caractérisé en ce que la couche de résine est constituée de plusieurs sortes de résines possédant des indices de réfraction différents afin d'obtenir différents effets dioptriques.

6. Procédé d'obtention selon la revendication 4 et 5 caractérisé en ce que les différentes couches de résine jointes possédant des indices de réfraction différents sont déposées de manière partielle, ponctuelle ou graduelle.

7. Procédé d' obtention selon la revendication 4, 5 et 6 caractérisé en ce que des lunettes des verres demontré sont incluses dans la couche de résine.

FIG. 1

FIG. 2

FIG. 3